# EUROPÄISCHE PATENTSCHRIFT

(11) **EP 3 619 249 B1**
(45) Veröffentlichungstag und Bekanntmachung des Hinweises auf die Patenterteilung: **22.03.2023**
(21) Anmeldenummer: 18719201.8
(22) Anmeldetag: 27.04.2018
(51) Int. Cl.: C08G 18/36, C08G 18/32, C08G 18/42, C08G 18/48, C08G 18/12, C08G 18/22, B01D 63/02, C02F 1/00, A61M 1/00, C02F 1/44, C08G 18/66

(54) **LAGERSTABILE POLYURETHANVERGUSSMASSE ZUR EINBETTUNG VON HOHLFASERN BEI DER HERSTELLUNG VON FILTERELEMENTEN**
STORAGE STABLE POLYURETHANE CASTING MASS FOR EMBEDDING OF HOLLOW FIBERS IN THE PRODUCTION OF FILTER ELEMENTS
MÉLANGE DE SCELLEMENT POLYURÉTHANE STABLE AU STOCKAGE DESTINÉ À ENROBER DES FIBRES CREUSES LORS DE LA FABRICATION D'ÉLÉMENTS DE FILTRE

(30) Priorität: 05.05.2017 EP 17169699
(43) Veröffentlichungstag der Anmeldung: 11.03.2020
(73) Patentinhaber: BASF SE, 67056 Ludwigshafen am Rhein (DE)
(72) Erfinder: BOLZE, Patrick, 49448 Lemfoerde (DE); KAMM, Andre, 49448 Lemfoerde (DE); MATHIEU, Thomas, 49448 Lemfoerde (DE); FRITZ, Ralf, 49448 Lemfoerde (DE); LUKAT, Gunther, 49448 Lemfoerde (DE)
(74) Vertreter: BASF IP Association
(86) Internationale Anmeldenummer: PCT/EP2018/060832
(87) Internationale Veröffentlichungsnummer: WO 2018/202566

(56) Entgegenhaltungen:
- DE-T2- 69 026 849

## Beschreibung

Die vorliegende Erfindung betrifft Polyurethanvergussmassen zur Einbettung von Hohlfasern von Filterelementen, erhältlich durch Vermischen einer Polyolkomponente (A) und einer Isocyanatkomponente (B), enthaltend mindestens ein aromatisches Isocyanat, zu einer Reaktionsmischung und Ausreagieren der Mischung zur Polyurethanvergussmasse, wobei die Polyolkomponente (A) mindestens ein fettsäurebasiertes Polyol (a1) mit einer Hydroxylzahl von größer 50 bis kleiner 500 mg KOH/g und einer Funktionalität von 2 bis 6, und mindestens einen Bismuthkatalysator (a2), erhältlich durch Vermischen von einem Bismuthcarboxylat (a2-I) mit einer Aminverbindung (a2-II) mit mindestens einem tertiären Stickstoffatom und mindestens einem gegenüber Isocyanat reaktivem Wasserstoffatom, wobei das Molverhältnis von Bismuth zu der Aminverbindung (a2-II) 1 zu 0,5 bis 1 zu 50 beträgt. Weiter betrifft die vorliegende Erfindung ein Verfahren zur Herstellung von Filterelementen unter Einsatz der Polyurethanvergussmassen und die Verwendung der Polyurethanvergussmassen zur Einbettung von Hohlfasern.

Die Verwendung von Polyurethanvergussmassen zur Einbettung von Hohlfasern von Filterelementen, insbesondere für Filter, die im medizinischen Bereich eingesetzt werden, wie die Verwendung als Einbettwerkstoff für Hohlfasern in Dialysatoren ist bekannt und beispielsweise in EP844015 B1 beschrieben. Der Vorteil der Polyurethanvergussmassen aus Polyurethan ist, dass es möglich ist, einen Volumenanteil an Hohlfasern in den Dialysator einzubauen und eine optimale Tränkung der Hohlfasern zu erreichen. Weiter ist das für die Herstellung der Filter beschriebene Polyurethan hydrolysestabil, übersteht das Verfahren der Heißdampfsterilisation unbeschadet und weist insbesondere kein toxisches Potential auf. Um allerdings eine gute und schnelle Aushärtung und eine hohe Produktivität zu erreichen, ist der Einsatz von Katalysatoren notwendig.

Üblicherweise werden die Rohstoffe zur Herstellung der Polyurethanvergussmassen vorgemischt, meist als Isocyanatkomponente und Polyolkomponente. Der Hersteller der Filterelemente muss dann die beiden Komponenten nur im geeigneten Verhältnis mischen, um die Polyurethanvergussmassen zu erhalten. Der Transport der Isocyanatkomponente und der Polyolkomponente zum Verarbeiter erfolgt in der Regel in Fässern oder Tankzügen. Zwischen der Herstellung der Komponenten und deren Verarbeitung vergehen dabei oft mehrere Tage bis Monate. Die Reaktivität der Polyol-Komponente mit der Isocyanat-Komponente sollte sich in diesem Zeitraum aber nicht ändern, damit ein gleichbleibender Viskositätsaufbau und dadurch eine gleichbleibende Tränkung der Hohlfasern sowie Aushärtung und Produktivität gewährleistet ist.

US 3962094 beschreibt den Einsatz katalysatorfreier Polyurethansysteme zur Herstellung der Polyurethanvergussmassen. Diese weisen zwar gleichbleibende Reaktivität auf, durch die geringe Reaktivität aber müssen lange Zykluszeiten und eine geringe Produktivität in Kauf genommen werden.

Der Einsatz von aminischen Katalysatoren, wie 1,4-Diazabicyclo[2.2.2]octan zur Herstellung von Polyurethanvergussmassen ist ebenfalls bekannt. Da diese Katalysatoren aber ein hohes Migrationsverhalten zeigen und aus dem erhaltenen Polyurethan austreten können, sind diese aber für die medizinische Anwendung nicht nutzbar. Um diesen Nachteil zu umgehen offenbart US 4224164 den Einsatz N,N,N',N'-tetrakis(2-hyroxypropyl)ethylendiamin. Dieser Katalysator weist gegenüber Isocyanat reaktive Gruppen auf und wird daher ins Polyurethannetzwerk integriert, so dass er nicht migrieren kann. Durch die katalytischen Eigenschaften dieses einbaubaren Katalysators können die Reaktivitäten deutlich gesteigert werden, jedoch können für die Industrie interessante Zykluszeiten erst mit Konzentrationen von größer 40 Gew.-% in der Polyolkomponente erhalten werden. Durch diesen hohen Anteil N,N,N',N'-tetrakis(2-hyroxypropyl)ethylendiamin steigt die Viskosität der Reaktionsmischung zur Herstellung der Polyurethanvergussmasse so stark an, dass eine optimale Tränkung der Hohlfasern nicht mehr gewährleistet ist.

DD-A-155777 offenbart den Einsatz von Zinnverbindungen als Katalysatoren für die Herstellung von Polyurethanvergussmassen für Dialysatoren. Die in DD-A-155777 beschriebenen Katalysatoren liefern eine hohe und konstante Reaktivität und haben sich, neben weiteren zinnorganischen Verbindungen, als Standard für PU-Polyurethanvergussmassen in der Industrie etabliert. Beispielsweise seinen hier genannt EP 2 081 973, EP 538 673, EP 413 265 und EP 329473. Aufgrund des toxischen Potentials von Organozinnverbindungen und der entsprechenden Einstufungen durch REACH, ist die Industrie auf der Suche nach neuen Katalysatoren. Jedoch zeigen alle gängigen Metallkatalysatoren in Polyolkomponenten, die für die erforderliche Hydrophobie nötigen Fettsäurepolyole, wie zum Beispiel Ricinusöl enthalten, keine ausreichende Lagerstabilität. Dies führt zu sich ändernden Reaktionszeiten bei der Aushärtung der Polyurethanvergussmassen in Abhängigkeit von der Lagerdauer. Dies wird von den Herstellern der Filterelemente nicht akzeptiert.

Die Verwendung von Polyurethanelastomeren als Dichtungsmaterial in elektrischen Geräten und als Material zum Bündeln der Enden bzw. Verbundmaterial für medizinische und industrielle Hohlfasern ist ebenfalls aus der EP 0 383 596 (DE 690 26 849 T2) bekannt. Die Polyurethanvergussmassen werden hierbei aus aliphatischen Diisocyanaten und Rizinusöl mit einer Hydroxylzahl von 120-160 mg KOH/g in Anwesenheit von N,N,N',N'-tetrakis (2-hydroxypropyl)ethylendiamin (THPED) oder N,N,N',N'-tetrakis (2-hydroxyethyl)ethylendiamins (THEED) hergestellt. Die hergestellen Polyurethanvergussmassen weisen ein geringen Gehalt an herauslösbaren Stoffen auf, entsprechend einer geringen Toxizität.

Aufgabe der vorliegenden Erfindung war es daher lagerstabile Komponenten zur Herstellung von Polyurethanvergussmassen zur Einbettung von Hohlfasern von Filterelementen zu liefern, wobei keine toxischen Materialien zum Einsatz kommen, aus der Polyurethanvergussmasse auch unter Sterilisationsbedingungen keine toxischen Materialien emittieren und eine konstant schnelle Reaktionszeit eingehalten werden kann. Dabei sollte das Aushärteverhalten idealerweise dem von gängigen Zinnkatalysatoren entsprechen.

Die vorliegende Aufgabe wurde gelöst durch Polyurethanvergussmassen zur Einbettung von Hohlfasern von Filterelementen, erhältlich durch Vermischen einer Polyolkomponente (A) und einer Isocyanatkomponente (B), enthaltend mindestens ein aromatisches Isocyanat, zu einer Reaktionsmischung und Ausreagieren der Mischung zur Polyurethanvergussmasse, wobei die Polyolkomponente (A) mindestens ein fettsäurebasiertes Polyol (a1) mit einer Hydroxylzahl von größer 50 bis kleiner 500 mg KOH/g und einer Funktionalität von 2 bis 6, und mindestens einen Bismuthkatalysator (a2), erhältlich durch Vermischen von einem Bismuthcarboxylat (a2-I) mit einer Aminverbindung (a2-II) mit mindestens einem tertiären Stickstoffatom und mindestens einem gegenüber Isocyanat reaktivem Wasserstoffatom, wobei das Molverhältnis von Bismuth zu der Aminverbindung (a2-II) 1 zu 0,5 bis 1 zu 50 beträgt.

Die erfindungsgemäßen Polyurethanvergussmassen sind vorzugsweise kompakt und werden vorzugsweise zum Einbetten von Hohlfasern für Filterelemente eingesetzt, das heißt, sie ist als Einbettungsmasse für Hohlfasern für Filterelemente geeignet. Unter kompakten Polyurethanvergussmassen werden Polyurethanvergussmassen mit einer Dichte von im Allgemeinen 0,8 g/l bis 1,3 g/l, bevorzugt von 0,9 g/l bis 1,1 g/l verstanden. Die erfindungsgemäßen Polyurethanvergussmassen zeigen nach erfolgter Aushärtung im allgemeinen eine Härte von 40 bis 80 Grad Shore-D. Bevorzugt weisen die erfindungsgemäßen Vergussmassen jedoch eine Härte von 55 bis 75 Grad Shore-D auf. Besonders bevorzugt, beispielsweise für Anwendungen als Vergussmasse in Dialysefiltern, ist eine Härte von 58 bis 70 Grad Shore-D. Die Härte in Grad Shore-D bezieht sich auf DIN 53505 bei einer Temperatur von 23°C. Der Fachmann wählt die Zusammensetzung der Vergussmassen, beispielsweise Art und Menge der hochfunktionellen Polyole, beispielsweise der Polyole (a3), entsprechend.

Bei der Herstellung der Filterelemente wird vorzugsweise die Polyolkomponente (A) und die Isocyanatkomponente (B) vermischt und in eine Form gegeben, die Hohlfasern enthält. Der Eintrag der Vergussmasse erfolgt hierbei besonders bevorzugt in einen in einer Zentrifuge rotierenden Hohlkörper, welcher Hohlfasern enthält. Durch Zentrifugalkraft wird das flüssige Reaktionsgemisch an die jeweiligen beiden Enden des Filterelements unter Umschließung der Hohlfasern transportiert und härtet zum kompakten, im wesentlichen klaren Verguss aus.

Der Aushärtungsschritt erfolgt wiederum ohne weiteres Zutun durch Reaktion der NCO-Gruppen mit reaktiven Wasserstoffatomen, insbesondere der OH-Gruppen, gegebenenfalls bei erhöhter Temperatur. Der Aushärtungsschritt ist abgeschlossen, sobald die Vergussmasse weitgehend ihre endgültigen Eigenschaften, insbesondere ihre Härte und ihre Stabilität, erreicht hat.

Durch einen anschließenden Schneidprozess werden üblicherweise die Öffnungen der Hohlfasern freigelegt. Das Filterelement ist im Allgemeinen nach einem Reinigungs- und Sterilisationsprozess einsatzbereit.

Durch das erfindungsgemäße Verfahren ist es möglich, Vergussmassen herzustellen, die heissdampfsterilisierbar und nicht zytotoxisch sind und beispielsweise bei der Trinkwassergewinnung oder -Reinigung als Wasserfilter oder im medizinisch-technischen Bereich beispielsweise als Dialysefilter eingesetzt werden können. Gleichzeitig können mir den erfindungsgemäßen Polyurethanvergussmassen komplexe Strukturen ausgeformt und beispielsweise eine hohe Faserzahl von mehr als 12.000 Hohlfasern pro Filter, wie in Dialysefilter erforderlich, vollständig umschlossen werden. Weiter sind die erfindungsgemäßen Polyurethanvergussmassen heißsterilisierbar oder nasssterilisierbar, beispielsweise unter Einsatz von Peressigsäure und zeigen keine Migration zytotoxischer Verbindungen, beispielsweise Aminverbindungen.

Die ausgehärteten Polyurethanvergussmassen sind resistent gegenüber Desinfektionsmitteln. Insbesondere zeigen die erfindungsgemäßen Vergussmassen eine geringe Aufnahme von Wasserdampf oder kochend heißem Wasser. Die erfindungsgemäßen Polyurethanvergussmassen lassen sich über einen Zeitraum von zwei Wochen ohne Bildung von Feinstaub schneiden, der andernfalls die Poren der für die eigentliche Filtration eingesetzten Hohlfasern verstopfen kann. Die erfindungsgemäßen, gehärteten Polyurethanvergussmassen sind vorzugsweise transparent, nicht zytotoxisch und besitzen vorzugsweise eine gute Haftung zu anderen Werkstoffen die Üblicherweise als Filtergehäuse dienen, z.B. Polycarbonaten, bei erhöhten Temperaturen über einen längeren Zeitraum. Die Polyurethanvergussmassen sind gegen Percarbonsäuren stabil, so dass Formkörper aus solchen Polyurethanvergussmassen mit z.B. Peressigsäure sterilisiert werden können. Die erfindungsgemäßen Polyurethanvergussmassen zeigen eine hohe Hydrophobie und eine ausreichende Vernetzungsdichte.

Die noch fließfähigen Polyurethanvergussmassen lassen sich auch ohne Schaumbildung vergießen. Gleichzeitig zeigen die erfindungsgemäßen Polyurethanvergussmassen unmittelbar nach Mischung der Reaktivkomponenten eine niedrige Mischviskosität. Die Polyurethanvergussmassen sind bereits nach 2 Stunden schneidbar, härten jedoch nicht so stark nach, so dass sie auch nach mehr als 24 Stunden noch schneidfähig sind. Vorteilhaft ist ferner, dass die erfindungsgemäßen Vergussmassen auf Basis von Polyurethan mit allen üblichen Hohlfaserarten, wie z.B. Cuprophan-, Polysulfon-, Polycarbonat- oder Cellulosefasern, verarbeitbar sind und die Polycarbonate, die häufig als Materialien des Filtergehäuses eingesetzt werden, vor der Verarbeitung keiner Vorbehandlung durch Corona-Entladung zur Verbesserung der Haftfestigkeit bedürfen.

Weiter betrifft die vorliegende Erfindung auch ein Verfahren zur Herstellung der erfindungsgemäßen Polyurethanvergussmassen. Dazu werden Polyolkomponente (A) und Isocyanatkomponente (B) zu einer Reaktionsmischung vermischt und zur Polyurethanvergussmasse ausreagieren lassen. Die Vermischung kann mechanisch mittels eines Rührers oder einer Rührschnecke oder unter hohem Druck im sogenannten Gegenstrominjektionsverfahren durchgeführt werden. Dabei wird im Rahmen der Erfindung die Mischung der Komponenten (A) und (B) bei Reaktionsumsätzen kleiner 90 %, bezogen auf die Isocyanatgruppen, als Reaktionsmischung bezeichnet.

Dabei enthält die Polyolkomponente (A) (a1) mindestens ein fettsäurebasiertes Polyol, vorzugsweise mit einer Hydroxylzahl von größer 50 bis kleiner 500 mg KOH/g und einer Funktionalität von mindestens 2, mindestens einen Bismuthkatalysator (a2), erhältlich durch Vermischen von einem Bismuthcarboxylat (a2-I) mit einer Aminverbindung (a2-II) mit mindestens einem tertiären Stickstoffatom und mindestens einem gegenüber Isocyanat reaktivem Wasserstoffatom, wobei das Molverhältnis von Bismuth zu der Aminverbindung (a2-II) 1 zu 0,5 bis 1 zu 50 beträgt.

Unter OH-Funktionalität ist dabei im Rahmen der vorliegenden Erfindung die Zahl an alkoholischen, acylierbaren OH-Gruppen pro Molekül zu verstehen. Falls die betreffende Komponente aus einer Verbindung mit definierter Molekularstruktur besteht, ergibt sich die Funktionalität aus der Zahl der OH-Gruppen pro Molekül. Wird eine Verbindung durch Ethoxylierung oder Propoxylierung eines Startermoleküls hergestellt, ergibt sich die OH-Funktionalität aus der Anzahl der reaktiven funktionellen Gruppen, beispielsweise OH-Gruppen, pro Startermolekül.

Als fettsäurebasiertes Polyol geeignet sind vorzugsweise solche mit einer Hydroxylzahl von größer 50 bis kleiner 500 mg KOH/g, besonders bevorzugt 100 bis 300 mg KOH/g und insbesondere 100 bis 200 mg KOH/g, und einer Funktionalität von mindestens 2. Die OH-Funktionalität der Fettsäurebasierten Polyole liegt bevorzugt im Bereich von 2 bis 3. Besonders bevorzugt liegt die OH-Funktionalität von der fettsäurebasierten Polyole bei 2,3 bis 3 und ganz besonders bevorzugt bei 2,6 bis 3.

Ein fettbasiertes Polyol kann ein Fett, ein Öl, eine Fettsäure oder ein Fettsäurederivat sein oder aus den vorgenannten Verbindungen durch physikalische oder chemische Modifikation erhalten werden. Fettbasierte Polyole nach oben genannter Definition sind dem Fachmann an sich bekannt oder können nach an sich bekannten Methoden erhalten werden.

Als fettbasiertes Polyol kommen beispielsweise pflanzliche Öle oder deren Derivate in Betracht. Pflanzliche Öle können in ihrer Zusammensetzung schwanken und in unterschiedlichen Reinheitsgraden vorkommen. Bevorzugt im Sinne dieser Erfindung sind pflanzliche Öle, die den Bestimmungen des Deutschen Arzneibuches (DAB) genügen. Ganz besonders bevorzugt enthält die Komponente a1) mindestens ein fettbasiertes Polyol, das ein pflanzliches Öl ist, welches DAB-10 entspricht.

Als fettbasiertes Polyol können außerdem allgemein bekannte Fettsäuren, bevorzugt natürliche Fettsäuren, besonders bevorzugt pflanzliche Fettsäuren, insbesondere ungesättigte pflanzliche Fettsäuren, als auch deren Derivate wie die Ester mit Mono-, Di-, und/oder Trialkoholen verwendet werden, sofern die weiteren Eigenschaften hinsichtlich Molekulargewicht und OH-Funktionalität erfüllt sind.

Als fettbasiertes Polyol können jedoch beispielsweise auch ringgeöffnete epoxidierte oder oxidierte Fettsäureverbindungen und/oder Addukte von Fettsäureverbindungen und Alkylenoxiden eingesetzt werden. Bevorzugt sind hydroxylierte Fettsäuren und/oder hydroxylierte Fettsäurederivate, die durch die vorgenannten Verfahren erhältlich sind.

Die Addukte von OH-funktionellen fettbasierten Verbindungen, beispielsweise Ricinusöl oder hydroxylierte pflanzliche Öle, und Alkylenoxiden können durch allgemein bekannte Alkoxylierung der Verbindungen mit beispielsweise Ethylenoxid, Propylenoxid und/oder Butylenoxid bei Temperaturen von 80 bis 130 °C und Drücken von 0,1 bis 1 MPa, gegebenenfalls in Gegenwart von üblichen Katalysatoren wie Alkalihydroxiden oder Alkalialkoholaten hergestellt werden.

Als fettbasiertes Polyol können des weiteren auch hydroxylierte Fettsäureverbindungen auf der Basis von Rapsöl, Sojaöl, Rüböl, Olivenöl und/oder Sonnenblumenöl und/oder solche auf der Basis von Öl- und/oder Linolsäure eingesetzt werden. Als fettbasiertes Polyol sind insbesondere Polyole geeignet, die auf hydroxyliertem Sojaöl basieren.

Bevorzugt sind außerdem Triglyceride von Fettsäuren, die eine OH-Funktionalität von 2 bis 3 aufweisen. Besonders bevorzugt sind das Triglycerid von Ricinolsäure, gegebenenfalls im Gemisch mit Triglyceriden, die noch weitere natürliche Fettsäuren enthalten, beispielsweise Linolsäure und/oder Palmitinsäure.

Besonders bevorzugt wird als fettbasiertes Polyol jedoch ein pflanzliches Öl ohne chemische Modifikation eingesetzt. Besonders bevorzugt ist Ricinusöl oder das Alkoxylierungsprodukt von Ricinusöl, insbesondere Ricinusöl. Insbesondere bevorzugt als fettbasiertes Polyol ist Ricinusöl, das den Bestimmungen des Deutschen Arzneibuches gemäß DAB 10 genügt. In einer besonders bevorzugten Ausführungsform wird als Komponente (a1) ausschließlich Ricinusöl eingesetzt.

Die Komponente (a1) weist bevorzugt einen geringen Wassergehalt, beispielsweise kleiner 0,2 Gew.-% auf. Bevorzugt ist ein Wassergehalt der Komponente (a1) von weniger als 0,1 Gew.-%. Wird als Komponente (a1) ein natürliches Öl, beispielsweise Ricinusöl, eingesetzt, dann erfolgt üblicherweise vor dem Einsatz eine Reinigung, die insbesondere die Entfernung von Schwebstoffen und eine Entwässerung einschließen kann. Von Schwebstoffen befreite natürliche Öle mit oben genanntem Wassergehalt sind als Komponente (a1) besonders geeignet.

Der Bismuthkatalysator (a2) ist erhältlich durch Vermischen von einem Bismuthcarboxylat (a2-I) mit einer Aminverbindung (a2-II) mit mindestens einem tertiären Stickstoffatom und mindestens einem gegenüber Isocyanat reaktivem Wasserstoffatom. Dabei beträgt das Molverhältnis von Bismuth zu der Aminverbindung (a2-II) 1 zu 0,5 bis 1 zu 50, vorzugsweise 1 zu 1 bis 1 zu 20, besonders bevorzugt 1 zu 1 bis 1 zu 10 und insbesondere 1 : 2 bis 1 : 5.

Bei den Bismutcarboxylaten (a2-I) liegt Bismut bevorzugt in den Oxidationsstufen 2 oder 3 vor, insbesondere 3. Zur Salzbildung werden als Carbonsäuren bevorzugt Carbonsäuren mit 6 bis 18 Kohlenstoffatomen, besonders bevorzugt mit 8 bis 12 Kohlenstoffatomen verwendet. Beispiele für besonders geeignete Bismutsalze sind Bismut(III)-neodecanoat, Bismut-2-etyhlhexanoat und Bismut-octanoat, besonders bevorzugt wird Bismut(III)-neodecanoat eingesetzt.

Zur Herstellung des erfindungsgemäßen Bismuthkatalysators wird das Bismuthcarboxylat (a2-I) mit der Aminverbindung (a2-II) , vorzugsweise bei einer Temperatur von 10 bis 120 °C, besonders bevorzugt 20 bis 100 °C und insbesondere 50 bis 80 °C, versetzt. Dabei wird die Temperatur vorzugsweise so gewählt, dass die Aminverbindung (a2-II) flüssig ist. Das Vermischen erfolgt vorzugsweise unter Rühren. In einer bevorzugten Ausführungsform wird die Mischung bei der Mischtemperatur mindestens weitere 5 Minuten, besonders bevorzugt 10 bis 120 Minuten und insbesondere 10 bis 60 Minuten gerührt und anschließend abkühlen lassen. Falls erforderlich kann noch ein Lösungsmittel zugegeben werden, beispielsweise Glycole, wie Diethylenglycol oder Monoethylenglcyol. Vorzugsweise werden der Mischung aus Bismuthcarboxylat (a2-I) und Aminverbindung (a2-II) bei der Herstellung des Bismuthkatalysators (a2) neben den Verbindungen (a2-I) und (a2-II) weniger als 100Gew-%, bevorzugt weniger als 50 Gew.-%, bezogen auf das Gesamtgewicht der Verbindungen (a2-I) und (a2-II), weitere Verbindungen zugegeben, insbesondere erfolgt die Herstellung des Bismuthkatalysators (a2) vor der Herstellung der Polyolkomponente (A) und damit vor der Zugabe der des Fettsäurebasierten Polyols (a1).

Als Aminverbindung (a2-II) kann mindestens eine Aminverbindung mit mindestens einem tertiären Stickstoffatom und mindestens einem gegenüber Isocyanat reaktivem Wasserstoffatom eingesetzt werden. Vorzugsweise weisen die Aminverbindungen mit mindestens einem tertiären Stickstoffatom eine Hydroxylzahl von 500 bis 1200 mg KOH/g und einer Isocyanatfunktionalität von mindestens 3, vorzugsweise 3 bis 8 und besonders bevorzugt 3 bis 6 auf. Das bedeutet, dass die Verbindung (a2) mindestens 3, vorzugsweise 3 bis 8 und besonders bevorzugt 3 bis 6 gegenüber Isocyanatgruppen reaktive Wasserstoffatome aufweist. DieVerbindungen (a2-II) werden vorzugsweise durch Alkoxylierung, vorzugsweise Ethoxylierung oder Propoxylierung von Aminen oder höherfunktionellen Aminen, beispielsweise Diaminen oder Triaminen erhalten. Als Starterverbindung kann beispielsweise Methylamin, Ethylamin, Isopropylamin, Butylamin, Benzylamin, Anilin, Toluidin, Toluoldiamin, Naphtylamin, Ethylendiamin, Diethylentriamin, 4,4'-Methylendianilin, 1,3-Propandiamin, 1,6-Hexandiamin, Ethanolamin, Diethanolamin, Triethanolamin Amoniak oder Ethylendiamin oder Mischungen davon eingesetzt werden. Dabei werden die Startermoleküle so ausgewählt, und in solchen Mengen eingesetzt, dass die mittleren nominalen Funktionalitäten erhalten werden. Als Nominale Funktionalität wird dabei im Rahmen dieser Erfindung die Funktionalität betrachtet, die sich alleine durch die Funktionalität und den Mengenanteil der Startermoleküle ergibt. Eine eventuelle Erniedrigung der Funktionalität, beispielsweise durch Nebenreaktionen, bleibt unberücksichtigt.

Aminverbindungen (a2-II) weisen vorzugsweise eine Hydroxylzahl von 600 bis 1100 mg KOH/g, besonders bevorzugt 650 bis 950 mg KOG/g auf. Besonders bevorzugt wird als Aminverbindung mit mindestens einem tertiären Stickstoffatom N,N,N',N'-tetrakis(2-hyroxypropyl)ethylenediamin, N,N,N',N'-tetrakis(2-hyroxyethyl)ethylendiamin oder Triisopropanolamin eingesetzt. In einer besonders bevorzugten Ausführungsform enthält die Komponente (a2) nur eine Aminverbindung mit mindestens einem tertiären Stickstoffatom, insbesondere N,N,N',N'-tetrakis(2-hyroxypropyl)ethylenediamin, N,N,N',N'-tetrakis(2-hyroxyethyl)ethylenediamin oder Triisopropanolamin.

In einer bevorzugten Ausführungsform werden zur Herstellung der erfindungsgemäßen Polyurethanvergussmassen keine organischen Zinnverbindungen eingesetzt, die als Katalysatoren für die Polyurethanreaktion bekannt sind. Weiter bevorzugt wird als Metallkatalysator ausschließlich ein Bismuthkatalysator eingesetzt. In einer ganz besonders bevorzugten Ausführungsform wird als Polyurethankatalysator ausschließlich der Bismuthkatalysator (a2) eingesetzt.

Solche Bismuthkatalysatoren werden beispielsweise in WO2016/114876 als Katalysatoren für wassergetriebene Polyurethanschäume offenbart und sind kommerziell als Bicat^{®} 8840 und Bicat^{®} 8842 der Firma Shepherd zu beziehen.

Neben den Komponenten (a1) und (a2) können in der Polyolkomponente (A) weitere Verbindungen enthalten sein, beispielsweise weitere Polyole (a3). Dabei können alle in der Polyurethanchemie bekannte Polyole eingesetzt werden, welche nicht unter die Definition der Komponente (a1) fallen.In einer bevorzugten Ausführungsform weises diese keine tertiären Stickstoffatome auf. Besonders bevorzugt enthält die Polyolkomponente (A) mindestens ein mindestens difunktionelles Polyol (a3) mit einer Funktionalität von 2 bis 8 und einer Hydroxylzahl von 600 bis 1350 mg KOH/g. Diese Polyole (a3) können durch Alkoxylierung, vorzugsweise Ethoxylierung oder Propoxylierung, von Di- oder höherfunktionellen Startermolekühlen, beispielsweise Ethylenglycol, Diethylenglycol, Propylenglycol, Dipropylenglycol, Glycerin, Trimethylolpropan, Pentaerythrit, Zuckerderivate, wie Saccharose, Hexitderivate, wie Sorbit sowie andere zwei oder mehrwertige Alkohole oder Mischungen davon erhalten werden. Dabei werden die Startermoleküle so ausgewählt, und in solchen Mengen eingesetzt, dass die mittleren nominalen Funktionalitäten erhalten werden. Als Nominale Funktionalität wird dabei im Rahmen dieser Erfindung die Funktionalität betrachtet, die sich alleine durch die Funktionalität und den Mengenanteil der Startermoleküle ergibt. Eine eventuelle Erniedrigung der Funktionalität, beispielsweise durch Nebenreaktionen, bleibt unberücksichtigt.

In einer weiteren Ausführungsform der Erfindung kann auch die Verbindung a2-II als weitere Komponente dem System zugesetzt werden. Bevorzugt wird allerdings die Verbindung a2-II der Polyolkomponente (A) nicht als weiteres Polyol zugesetzt.

Vorzugsweise werden die Komponenten (a1) bis (a3) in einer Menge eingesetzt, dass der Anteil des fettsäurebasierten Polyols (a1) 60 bis 99 Gew.-%, vorzugsweise 75 bis 98 Gew.-% und besonders bevorzugt 90 bis 96 Gew.-%, der Anteil des Bismuthkatalysators (a2) 0,001 bis 2,0 Gew.-%, vorzugsweise 0,001 bis 1 Gew.-5, mehr bevorzugt 0,01 bis 0,8 und besonders bevorzugt 0,02 bis 0,4 Gew.-% und der Anteil des Polyols (a3) 0 bis 25 Gew.-%, vorzugsweise 0,5 bis 10 und besonders bevorzugt 1,0 bis 5 Gew.-% jeweils bezogen auf das Gesamtgewicht der Komponenten (a1) bis (a3), beträgt. Besonders bevorzugt enthält die Polyolkomponente (A) neben den Komponenten (a1) bis (a3) weniger als 20 Gew.-%, besonders bevorzugt weniger als 10 Gew.-% und insbesondere weniger als 5 Gew.-%, jeweils bezogen auf das Gesamtgewicht der Komponenten (a1) bis (a3) weitere Verbindungen. In einer ganz bevorzugten Ausführungsform enthält die Polyolkomponente (A) neben den Verbindungen (a1) bis (a3) keine weiteren Verbindungen.

Als Isocyanatkomponente (B) können alle in der Polyurethanchemie bekannten, aromatischen Diisocyanate und höherfunktionellen Isocyanate eingesetzt werden. Vorzugsweise enthält die zur Herstellung der erfindungsgemäßen Polyurethanvergussmassen Isocyanatprepolymere. Solche Isocyanatprepolymere werden durch Reaktion von Diisocyanaten und höherfunktionellen Isocyanaten (b1) mit Verbindungen mit gegenüber Isocyanaten reaktiven Gruppen (b2), vorzugsweise Diolen, erhalten, wobei Isocyanate im Überschuss eingesetzt werden.

Als Isocyanat-Komponente b1) kommen die üblichen aromatischen Di- und/oder Polyisocyanate oder deren Mischungen zum Einsatz. Aromatische Isocyanate sind dabei im Rahmen der vorliegenden Erfindung Isocyanate, wobei eine Isocyanatgruppe unmittelbar an ein Kohlenstoffatom in einem Aromaten gebunden ist. Insbesondere geeignet sind Diisocyanate, beispielsweise Toluylendiisocyanat (TDI). Bevorzugt werden Diphenylmethandiisocyanate (nachfolgend als MDI bezeichnet). Sofern MDI verwendet wird, können alle 2-Kern-Isomere (2,2'; 2,4' und 4,4'), gegebenenfalls in Mischung mit höherkernigen Homologen des Diphenylmethandiisocyanats, verwendet werden.

Die Isocyanat-Komponente b1) kann zusätzlich modifiziert vorliegen, beispielsweise durch Einbau von Uretdion-, Carbamat-, Isocyanurat-, Carbodiimid-, Allophanatgruppen. Bevorzugt enthält die Komponente b1) 2 bis 10 Gew.-% eines Carbodiimid-modifizierten Isocyanates. Besonders bevorzugt ist hierbei ein Carbodiimid-modifiziertes 4,4'-MDI. Ganz besonders bevorzugt enthält die Isocyanat-Komponente b1) 3 bis 7 Gew.-% Carbodiimid-modifiziertes 4,4'-MDI. Die angegebenen Zahlenwerte in Gew.-% Carbodiimid-modifiziertes Isocyanat beziehen sich auf ein Carbodiimid-modifiziertes Isocyanat, welches 10 Gew.-% Carbodiimid enthält. Bei abweichendem Carbodiimidgehalt rechnet der Fachmann die angegebenen Werte entsprechend um.

Als Diol-Komponente b2) kommen organische Polyhydroxyverbindungen mit einer OH-Funktionalität von 1,5 bis 2,5 zum Einsatz. Bevorzugt liegt die OH-Funktionalität im Bereich von 1,8 bis 2,2, besonders bevorzugt wird eine Diol-Verbindung mit einer OH-Funktionalität von 2 eingesetzt. Als Diol-Komponente b2) bevorzugt werden insbesondere alkoxylierte Diol-Verbindungen. Besonders bevorzugt werden als Diol-Komponente b2) Propylenglykole.

Zu den geeigneten Propylenglykolen zählen (Mono-)Propylenglykol und Dipropylenglykol sowie Oligo- und Polypropylenglykole, wobei letztere ausgehend von einer Diolverbindung durch Propoxylierung hergestellt werden können.

Bevorzugt hat das modifizierte Isocyanat (B) einen NCO-Gehalt von 12 bis 30 Gew.-%, besonders bevorzugt von 18 bis 27 Gew.-% und insbesondere von 20 bis 25 Gew.-%.

Weiter können zur Herstellung der erfindungsgemäßen Polyurethanvergussmassen Hilfs- und/oder Zusatzstoffen, wie z.B. Zellreglern, Trennmitteln, Pigmenten, Flammschutzmittel, Verstärkungsstoffen wie Glasfasern, oberflächenaktiven Verbindungen und/oder Stabilisatoren gegen oxidativen, thermischen, hydrolytischen oder mikrobiellen Abbau oder Alterung eingesetzt werden. Diese werden vorzugsweise der Polyolkomponente (A) zugegeben.

Die Umsetzung zur erfindungsgemäßen Polyurethanvergussmasse erfolgt bevorzugt ohne Zusatz von Treibmittel, so dass es sich bei dem erfindungsgemäßen Polyurethan um ein kompakte Polyurethan handelt. Dabei können die verwendeten Polyole (a) jedoch geringe Anteile an Restwasser enthalten. Bevorzugt ist der Restwassergehalt unter 1 Gew.-%, mehr bevorzugt unter 0,3 Gew.-%, besonders bevorzugt unter 0,05 Gew.-%, bezogen auf das Gesamtgewicht der eingesetzten Komponente (A). In einer weiteren Ausführungsform der Erfindung werden den der Polyolkomponente (A) noch übliche Wasserfänger zugesetzt. Werden diese zugesetzt ist deren Anteil < 20 Gew.-%, bevorzugt < 10 Gew-% und ganz besonders bevorzugt < 5 Gew.-%, bezogen auf das Gesamtgewicht der Komponente (A). Besonders bevorzugt wird allerdings ohne Wasserfänger gearbeitet.

Ferner werden bevorzugt die Einsatzstoffe so gewählt, dass die resultierenden Polyurethanvergussmassen heißdampfsterilisierbar und nicht zytotoxisch sind. Dies wird im Wesentlichen dadurch bewirkt, dass alle Einsatzstoffe so aufgebaut sind, dass sie entweder in das Polyurethan-Polymergitter eingebaut werden oder aus dem Polymer nicht mehr migrieren können bzw. das feste Polymere so hydrolysestabil ist, dass keine niedermolekularen Abbauprodukte, die zytotoxisch sein können, entstehen können.

Zur Herstellung der erfindungsgemäßen Polyurethane werden im allgemeinen die Komponenten (a) und (b) in solchen Mengen zur Umsetzung gebracht, dass das Äquivalenzverhältnis von NCO-Gruppen zur Summe der reaktiven Wasserstoffatome 1:0,8 bis 1:1,25, vorzugsweise 1:0,9 bis 1:1,15 beträgt. Ein Verhältnis von 1:1 entspricht hierbei einem NCO-Index von 100.

Die Ausgangskomponenten werden üblicherweise bei einer Temperatur von 0°C bis 100°C, bevorzugt 15 bis 70°C gemischt und zur Reaktion gebracht. Die Vermischung kann mit den üblichen PUR-Verarbeitungsmaschinen erfolgen. In einer bevorzugten Ausführungsform erfolgt die Vermischung durch Niederdruckmaschinen oder Hochdruckmaschinen. Anschließend werden die Vergussmassen vergossen und der Aushärtung unterzogen, beispielsweise bei Temperaturen von 20 bis 150 °C, vorzugsweise 40 bis 100 °C.

Unter Vergießen soll jede Maßnahme verstanden werden, die der anfänglich fließfähigen Vergussmasse diejenige Gestalt gibt, die sie nach dem Aushärten hat. Unter Vergießen soll insbesondere das Einbringen in oder das Aufbringen auf einen Körper verstanden werden. Bei einem solchen Körper kann es sich beispielsweise um eine Fläche, ein Rahmen, ein Gefäß mit mindestens einer Öffnung oder um eine Form mit mindestens einer Vertiefung handeln. Die Vergussmasse kann grundsätzlich in Kontakt mit dem Körper bleiben oder aus diesem herausgelöst werden. Bevorzugt wird die Vergussmasse nach erfolgter Aushärtung nicht von der Form getrennt, sondern bildet mit dieser eine Einheit.

Die erfindungsgemäßen Polyurethanvergussmassen werden zur Herstellung zum Verguss von Filterelementen eingesetzt. Dazu wird ein Bündel von Hohlfasern an deren Ende in einer erfindungsgemäßen Polyurethanvergussmasse eingebettet. Ein solches Filterelement kann als Wasserfilter, beispielsweise zur Gewinnung oder Aufbereitung von Trinkwasser, oder im medizinischen Bereich, beispielsweise als Dialysefilterelement eingesetzt werden.

Die erfindungsgemäßen Vergussmassen zeichnen sich durch ihre gute Bioverträglichkeit und eine geringe Emission von Stoffen, insbesondere von toxischen Substanzen, ihre schnelle und konstante Aushärtung und ihre gute Sterilisierbarkeit aus.

Im Folgenden soll die Erfindung anhand von Beispielen verdeutlicht werden.

### Eingesetzte Rohstoffe

| | |
|---|---|
| Poly 1: | Ricinusöl DAB von Alberdingk Boley |
| Poly 2: | Polyetherol auf Basis von Trimethylolpropan und Propylenoxid mit einer OH-Zahl von 935 mg KOH/g |
| Poly 3: | Polyetherol auf Basis von Trimethyolpropan und Propylenoxid mit einer OH-Zahl von 160 mg KOH/g |
| Poly 4: | Oleochemischer Polyester (Sovermol 819) mit einer OH-Zahl von 240 mg KOH/g |
| Poly 5: | Polyetherol auf Basis von Gylcerin und Propylenoxid mit einer OH-Zahl von 805 mg KOH/g |
| Poly 6: | Triisopropanolamin |
| Poly 7: | N,N,N',N'-tetrakis(2-hyroxypropyl)ethylendiamin |
| KV1: | Oxydipropanol |
| KV2: | 2-Methyl-1,3-propandiol |
| KV3: | Monoethylenglycol |
| ISO1: | Isocyanatprepolymer auf Basis von MDI, Dipropylenglycol und Polypropylenglycol mit einem NCO-Gehalt von 23 Gew.-% |
| ISO2: | Isocyanatprepolymer auf Basis von MDI, Dipropylenglycol , Polypropylenglycol und Rizinusöl mit einem NCO-Gehalt von 20,6 Gew.-% |
| Kat 1: | Zinnkatalysator Tinstab OTS 16 der Firma Akcros |
| Kat 2: | Bismuthkatalysator Coscat 83 |
| Kat 3: | Bismuthkatalysator Bicat 8118M der Firma Shepherd |
| Kat 4: | Bismuthkatalysator Bi 2010L der Firma Umicore |
| Kat 5: | Bismuthkatalysator Bicat 8840 der Firma Shepherd, hergestellt aus Bismuthneodecanoat und N,N,N',N'-Tetrakis(2-hydroxypropyl)ethylendiamin |
| Kat 6: | Bismuthkatalysator Bicat 8842 der Firma Shepherd, hergestellt aus Bismuthneodecanoat und N,N,N',N'-Tetrakis(2-hydroxyethyl)ethylendiamin |

Zur Bestimmung der Lagerstabilität der Polyolmischung wurden Polyole und Katalysatoren wie in den Tabellen 1 bis 3 angegeben zu einer Polyolkomponente vermischt, alle Angaben in den Tabellen entsprechen Gewichtsteilen, wenn nicht anders angegeben. Die erhaltenen Polyolkomponenten wurden wie angegeben bei Raumtemperatur unter Luftabschluss in einem verschlossenen Behältnis gelagert. Vor der Probenentnahme wurde die Mischung homogenisiert und danach entgast. Die erste Messung zur Bestimmung der Startreaktivität wurde 24 Stunden nach dem Ansetzen der Polyolmischung durchgeführt, damit das System sich einpendeln konnte. Danach wurden in unterschiedlichen Abständen die Gelzeit bestimmt.

Dabei erfolgte die Bestimmung der Gelzeit wie folgt. Einer entsprechenden Menge Polyolmischung wurde die benötigte Menge an Isocyanat bei einem Isocyanatindex von 105 zugesetzt. Die Mengen der Isocyanatkomponente und der Polyolkomponente wurden dabei so gewählt, dass 100 g Reaktionsmischung erhalten wurden. Die Reaktionsmischung wurde in einem SpeedmixerTM Becher PP130 bei 25°C 30 s bei 1800 RPM mittels eines SpeedmixerTM der Firma Haunschild gemischt und gleichzeitig die Messung an einen SHYODU Gel Timer gestartet. Nach den 30 Sekunden Mischzeit wurde der PP130 Becher unter dem Gel Timer platziert und die Gelzeit bestimmt. Dabei wird die Gelzeit als die Zeit bestimmt, in der die Viskosität der Reaktionsmischung bei konstanter Temperatur sich soweit erhöht, dass die erforderliche Rührkraft die gegebene Rührkraft des Shyodu Gel Timers übersteigt.

Die nachfolgenden Beispiele sollen den Effekt der erfindungsgemäßen Zusammensetzung verdeutlichen

**Tabelle 1**

| | V1 | V2 | V3 | V4 |
|---|---|---|---|---|
| Poly 1 | 100 | 95,0 | 90,0 | 85,0 |
| Poly 2 | | | | |
| Poly 7 | | 5,0 | 10,0 | 15,0 |
| Kat 1 | | | | |
| | | | | |
| Iso 1 | X | X | X | X |
| Iso 2 | | | | |
| Härte [Shore D] | 18 | 56 | 70 | 76 |
| Gelzeit [hh:mm:ss] | | | | |
| 1d | 01:14:06 | 00:38:11 | 00:19:10 | 00:08:16 |
| 14d | | | | |

Wie aus den Vergleichsbeispielen V1 bis V4 ersichtlich ist, führt der Einsatz von N,N,N',N'-tetrakis(2-hyroxypropyl)ethylendiamin zu einer Verkürzung der offenen Zeit und somit zu einer Steigerung der Reaktivität. Schnelle Zykluszeiten können allerdings nur mit hohen Konzentrationen von N,N,N',N'-tetrakis(2-hyroxypropyl)ethylendiamin erreicht werden. Dies hat allerdings den Nachteil, dass die Systeme deutlich an Härte gewinnen und dadurch die Schneidbarkeit der Systeme leidet.

**Tabelle 2**

| | V5 | V6 | V7 | V8 | V9 | V10 | V11 |
|---|---|---|---|---|---|---|---|
| Poly 1 | 94,92 | 94,9 | | 94,9 | | 94,9 | |
| Poly 2 | 5,00 | 5,0 | 5,04 | 5,0 | 5,04 | 5,0 | 5,04 |
| Poly 3 | | | 94,91 | | 94,91 | | 94,95 |
| Kat 1 | 0,08 | | | | | | 0,01 |
| Kat 2 | | 0,1 | 0,05 | | | | |
| Kat 3 | | | | 0,1 | 0,05 | | |
| Kat 4 | | | | | | 0,1 | |
| | | | | | | | |
| Iso 1 | | X | X | | X | | X |
| Iso 2 | X | | | X | | X | |
| Mischungsverhältnis 100: X | 76,9 | 69,1 | 69,8 | 76,9 | 69,8 | 76,9 | 69,9 |
| | | | | | | | |

| Gelzeit [mm:ss] | | | | | | | |
|---|---|---|---|---|---|---|---|
| 1d | 03:20 | 03:29 | 00:48 | 07:10 | 01:05 | 07:20 | 05:20 |
| 7d | n.m. | n.m. | 00:51 | n.m. | 01:05 | 11:00 | n.m. |
| 14d | n.m. | 04:48 | n.m. | n.m. | n.m. | n.m. | n.m. |
| 56d | 3:05 | 08:45 | 00:58 | 11:30 | 01:20 | >16:00 | 05:30 |
| | | | | | | | |

Wie aus den Vergleichsbeispielen V5 & V11 ersichtlich ist, führt die Verwendung von Zinnkatalysatoren in kompakten Polyurethansystemen zu lagerstabilen Mischungen mit gleichbleibender Reaktivität, unabhängig davon ob oleochemische Polyole im System verwendet werden. Die Beispiele V7 und V9 zeigen deutlich, dass Bismuthkatalysatoren in kompakten Polyurethansystemen, welche keine oleochemischen Polyole enthalten, als geeigneter Ersatz für Zinnkatalysatoren genutzt werden können. Die Reaktivität der Systeme bleibt auch hier nahezu konstant. Die Vergleichsbeispiele V6, V8 & V10 offenbaren, dass die Reaktivität der Systeme, welche Fettsäurepolyole enthalten, über die Zeit stark abbauen und Bismuthcarboxylate nicht als geeigneter Ersatz für Zinnkatalysatoren in solchen Systemen verwendet werden können.

**Tabelle 3**

| | B1 | B2 | B3 | B4 |
|---|---|---|---|---|
| Poly 1 | 94,90 | 94,80 | 89,27 | 15,00 |
| Poly 2 | 5,00 | 5,00 | 2,78 | |
| Poly 4 | | | | 78,35 |
| Poly 5 | | | | 4,50 |
| KV 1 | | | 7,87 | |
| KV 2 | | | | 2,00 |
| Kat 5 | | 0,20 | | |
| Kat 6 | 0,10 | | 0,09 | 0,15 |
| | | | | |
| Iso 1 | X | | X | X |
| Iso 2 | | X | | |
| Mischungsverhältnis 100: X | 69,2 | 76,8 | 81,8 | 96,5 |
| | | | | |

| Gelzeit [mm:ss] | | | | |
|---|---|---|---|---|
| 1d | 04:53 | 03:20 | 03:10 | 04:27 |
| 28d | n.m. | 03:23 | 03:21 | 04:10 |
| 56d | 04:41 | n.m. | 03:12 | 04:37 |

Die Beispiele B1 bis B4 zeigen, dass durch den Einsatz eines Katalysators auf Basis von Bismuthcarboxylaten und Alkanolaminen lagerstabile Mischungen mit oleochemischen Polyolen erhalten werden können.

Neben N,N,N',N'-tetrakis(2-hyroxypropyl)ethylendiamin und N,N,N',N'-tetrakis(2-hyroxyethyl)ethylendiamin können auch andere Alkanolamine für die Herstellung stabiler Bismuthkatalysatoren verwendet werden. Dies soll durch das nachfolgende Beispiel erläutert werden.

### Beispiel B5:

In einen 250 mL 4 Halskolben wurden mit Thermometer, Rührer, Kühler und Stickstoffeinleitung 75,00 g Kat 3 vorgelegt und auf 60°C erwärmt. Dem Katalysator wurden dann unter Rühren 44,23g geschmolzenesPoly 6 langsam zugeben. Nach Ende der Zugabe wurde das Gemisch noch für weitere 15 Minuten gerührt und 30 g KV 3 zugegeben. Die Mischung wurde dann abgefüllt und für weitere Versuche verwendet. Diese Umsetzungsprodukte zeigten ebenfalls eine gute Lagerstabilität und konstante Reaktivität wie das beigefügte Beispiel B6 zeigt:

**Tabelle 3**

| | B6 |
|---|---|
| Poly 1 | 94,90 |
| Poly 2 | 5,00 |
| Kat aus Beispiel B5 | 0,10 |
| | |
| Iso 1 | X |
| Mischungsverhältnis 100: X | 69,2 |
| | |

| Gelzeit [mm:ss] | |
|---|---|
| 1d | 06:21 |
| 14d | 06:18 |
| 90d | 06:12 |

## Patentansprüche

1. Polyurethanvergussmasse zur Einbettung von Hohlfasern von Filterelementen, erhältlich durch Vermischen einer Polyolkomponente (A) und einer Isocyanatkomponente (B) zu einer Reaktionsmischung und Ausreagieren der Mischung zur Polyurethanvergussmasse,
wobei die Polyolkomponente (A)
(a1) mindestens ein fettsäurebasiertes Polyol mit einer Hydroxylzahl von größer 50 bis kleiner 500 mg KOH/g und einer Funktionalität von 2 bis 6 und
(a2) mindestens einen Bismuthkatalysator, erhältlich durch Vermischen eines Bismuthcarboxylats (a2-I) mit einer Aminverbindung (a2-II) mit mindestens einem tertiären Stickstoffatom und mindestens einem gegenüber Isocyanat reaktivem Wasserstoffatom, wobei das Molverhältnis von Bismuth zu der Aminverbindung (a2-II) 1 zu 0,5 bis 1 zu 50 beträgt, enthält.

2. Polyurethanvergussmasse nach Anspruch 1, **dadurch gekennzeichnet, dass** die Aminverbindung (a2-II) mindestens drei gegenüber Isocyanat reaktive Wasserstoffatome aufweist.

3. Polyurethanvergussmasse nach einem der Ansprüche 1 oder 2, **dadurch gekennzeichnet, dass** die Aminverbindung (a2-II) ein alkoxyliertes Amin ist mit einer Hydroxylzahl von 500 bis 1200 mg KOH/g, die 3 bis 6 gegenüber Isocyanatgruppen reaktive Wasserstoffatome aufweist.

4. Polyurethanvergussmasse nach Anspruch 3, **dadurch gekennzeichnet, dass** die Aminverbindung (a2-II) ein Diamin gestartetes Propylenoxid mit einer nominalen Funktionalität von 3 bis 6 und einer Hydroxylzahl von 500 bis 900 mg KOH/g ist.

5. Polyurethanvergussmasse nach einem der Ansprüche 1 bis 4, **dadurch gekennzeichnet, dass** das fettbasierte Polyol (a1) Ricinusöl oder das Alkoxylierungsprodukt von Ricinusöl enthält.

6. Polyurethanvergussmasse nach einem der Ansprüche 1 bis 5, **dadurch gekennzeichnet, dass** die Polyolkomponente (A) mindestens ein mindestens difunktionelles Polyol (a3) mit einer Funktionalität von 2 bis 8 und einer Hydroxylzahl von 600 bis 1350 mg KOH/g enthält, das kein tertiäres Stickstoffatom aufweist.

7. Polyurethanvergussmasse nach einem der Ansprüche 1 bis 6, **dadurch gekennzeichnet, dass** der Anteil des fettsäurebasierten Polyols (a1) 60 bis 98 Gew.-%, der Anteil des Bismuthkatalysators (a2) 0,001 bis 1,0 Gew.-% und der Anteil des Polyols (a3) 0 bis 25 Gew.-%, jeweils bezogen auf das Gesamtgewicht der Komponenten (a1) bis (a3), beträgt.

8. Polyurethanvergussmasse nach einem der Ansprüche 1 bis 7, **dadurch gekennzeichnet, dass** die Isocyanatkomponente (B) Prepolymere von Isomeren und/oder Homologen des Diphenylmethandiisocyanats enthält.

9. Verfahren zur Herstellung von Filterelementen bei dem man ein Bündel von Hohlfasern an deren Ende in einer Polyurethanvergussmasse gemäß einem der Ansprüche 1 bis 8 einbettet und aushärtet.

10. Verfahren nach Anspruch 9, **dadurch gekennzeichnet, dass** das Filterelement ein Filterelement zum Einsatz in der Medizin ist.

11. Verfahren nach Anspruch 10, **dadurch gekennzeichnet, dass** das Filterelement ein Dialysefilterelement ist.

12. Verfahren nach Anspruch 9, **dadurch gekennzeichnet, dass** das Filterelement ein Wasserfilterelement ist.

13. Verwendung einer Polyurethanvergussmasse gemäß einem der Ansprüche 1 bis 8 zur teilweisen Einbettung von Hohlfasern.

## Claims

1. A polyurethane encapsulating compound for embedding hollow fibers of filter elements, obtainable by mixing a polyol component (A) and an isocyanate component (B) to give a reaction mixture and reacting the mixture to completion to give the polyurethane encapsulating compound,
wherein the polyol component (A) comprises
(a1) at least one fatty-acid-based polyol having a hydroxyl number of greater than 50 to less than 500 mg KOH/g and a functionality of from 2 to 6 and
(a2) at least one bismuth catalyst, obtainable by mixing a bismuth carboxylate (a2-I) with an amine compound (a2-II) having at least one tertiary nitrogen atom and at least one isocyanate-reactive hydrogen atom, wherein the molar ratio of bismuth to the amine compound (a2-II) is 1:0.5 to 1:50.

2. The polyurethane encapsulating compound according to claim 1, wherein the amine compound (a2-II) has at least three isocyanate-reactive hydrogen atoms.

3. The polyurethane encapsulating compound according to either of claims 1 and 2, wherein the amine compound (a2-II) is an alkoxylated amine having a hydroxyl number of from 500 to 1200 mg KOH/g and having 3 to 6 hydrogen atoms that are reactive toward isocyanate groups.

4. The polyurethane encapsulating compound according to claim 3, wherein the amine compound (a2-II) is a diamine-started propylene oxide having a nominal functionality of from 3 to 6 and a hydroxyl number of from 500 to 900 mg KOH/g.

5. The polyurethane encapsulating compound according to any of claims 1 to 4, wherein the fat-based polyol (a1) comprises castor oil or the alkoxylation product of castor oil.

6. The polyurethane encapsulating compound according to any of claims 1 to 5, wherein the polyol component (A) comprises at least one, at least difunctional, polyol (a3) which has a functionality of from 2 to 8 and a hydroxyl number of from 600 to 1350 mg KOH/g and does not have a tertiary nitrogen atom.

7. The polyurethane encapsulating compound according to any of claims 1 to 6, wherein the proportion of the fatty-acid-based polyol (a1) is 60 to 98% by weight, the proportion of the bismuth catalyst (a2) is 0.001 to 1.0% by weight and the proportion of the polyol (a3) is 0 to 25% by weight, based in each case on the total weight of components (a1) to (a3).

8. The polyurethane encapsulating compound according to any of claims 1 to 7, wherein the isocyanate component (B) comprises prepolymers of isomers and/or homologs of diphenylmethane diisocyanate.

9. A method for producing filter elements in which a bundle of hollow fibers is embedded and cured at their end in a polyurethane encapsulating compound according to any of claims 1 to 8.

10. The method according to claim 9, wherein the filter element is a filter element for use in medicine.

11. The method according to claim 10, wherein the filter element is a dialysis filter element.

12. The method according to claim 9, wherein the filter element is a water filter element.

13. The use of a polyurethane encapsulating compound according to any of claims 1 to 8 for the partial embedding of hollow fibers.

## Revendications

1. Masse de scellement de polyuréthane destinée à enrober des fibres creuses d'éléments filtrants, pouvant être obtenue par mélange d'un composant polyol (A) et d'un composant isocyanate (B) pour former un mélange réactionnel et par réaction du mélange pour obtenir une masse de scellement de polyuréthane,
le composant polyol (A) contenant
(a1) au moins un polyol à base d'acide gras, ayant un indice d'hydroxyle supérieur à 50 et inférieur à 500 mg de KOH/g et une fonctionnalité de 2 à 6 et
(a2) au moins un catalyseur à base de bismuth, pouvant être obtenu par mélange d'un carboxylate de bismuth (a2-I) avec un composé amine (a2-II) comportant au moins un atome d'azote tertiaire et au moins un atome d'hydrogène réactif vis-à-vis des isocyanates, le rapport en moles du bismuth sur le composé amine (a2-II) étant de 1:0,5 à 1:50.

2. Masse de scellement de polyuréthane selon la revendication 1, **caractérisée en ce que** le composé amine (a2-II) comprend au moins trois atomes d'hydrogène réactifs vis-à-vis des isocyanates.

3. Masse de scellement de polyuréthane selon l'une des revendications 1 ou 2, **caractérisée en ce que** le composé amine (a2-II) est une amine alcoxylée ayant un indice d'hydroxyle de 500 à 1 200 mg de KOH/g, qui comprend 3 à 6 atomes d'hydrogène réactifs vis-à-vis des groupes isocyanate.

4. Masse de scellement de polyuréthane selon la revendication 3, **caractérisée en ce que** le composé amine (a2-II) est un oxyde de propylène amorcé par une diamine, ayant une fonctionnalité nominale de 3 à 6 et un indice d'hydroxyle de 500 à 900 mg de KOH/g.

5. Masse de scellement de polyuréthane selon l'une des revendications 1 à 4, **caractérisée en ce que** le polyol (a1) à base de graisse est une huile de ricin ou contient un produit d'alcoxylation d'huile de ricin.

6. Masse de scellement de polyuréthane selon l'une des revendications 1 à 5, **caractérisée en ce que** le composant polyol (A) contient au moins un polyol au moins difonctionnel (a3) ayant une fonctionnalité de 2 à 8 et un indice d'hydroxyle de 600 à 1 350 mg de KOH/g, qui ne comprend pas d'atome d'azote tertiaire.

7. Masse de scellement de polyuréthane selon l'une des revendications 1 à 6, **caractérisée en ce que** la proportion du polyol (a1) à base d'acide gras est de 60 à 98 % en poids, la proportion du catalyseur à base de bismuth (a2) est de 0,001 à 1,0 % en poids et la proportion du polyol (a3) est de 0 à 25 % en poids, chacun par rapport au poids total des composants (a1) à (a3) .

8. Masse de scellement de polyuréthane selon l'une des revendications 1 à 7, **caractérisée en ce que** le composant isocyanate (B) contient des prépolymères d'isomères et/ou d'homologues du diisocyanate de diphénylméthane.

9. Procédé de fabrication d'éléments filtrants, dans lequel un faisceau de fibres creuses est incorporé et durci au niveau de leurs extrémités dans une masse de scellement de polyuréthane selon l'une des revendications 1 à 8.

10. Procédé selon la revendication 9, **caractérisé en ce que** l'élément filtrant est un élément filtrant pour utilisation en médecine.

11. Procédé selon la revendication 10, **caractérisé en ce que** l'élément filtrant est un élément filtrant de dialyse.

12. Procédé selon la revendication 9, **caractérisé en ce que** l'élément filtrant est un élément filtrant de l'eau.

13. Utilisation d'une masse de scellement de polyuréthane selon l'une des revendications 1 à 8 pour l'incorporation partielle de fibres creuses.
